# EUROPEAN PATENT SPECIFICATION

(11) **EP 1 660 461 B1**
(45) Date of publication and mention of the grant of the patent: **22.10.2008**
(21) Application number: 04775333.0
(22) Date of filing: 25.08.2004
(51) Int. Cl.: C07D 241/44, C07D 471/04, C07D 409/12, A61K 31/498, A61K 31/4985, A61P 11/00, A61P 19/00, A61P 25/00, A61P 29/00

(54) **NOVEL CONDENSED N-PYRAZINYL-SULPHONAMIDES AND THEIR USE IN THE TREATMENT OF CHEMOKINE MEDIATED DISEASES**
NEUE KONDENSIERTE N-PYRAZINYL-SULFONAMIDE UND DEREN VERWENDUNG BEI DER BEHANDLUNG VON CHEMOKINVERMITTELTEN KRANKHEITEN
NOUVEAUX N-PYRAZINYL-SULFAMIDES CONDENSES ET LEUR UTILISATION DANS LE TRAITEMENT DE MALADIES DONT LA MEDIATION EST ASSUREE PAR LES CHIMIOKINES

(30) Priority: 27.08.2003 SE 0302304
(43) Date of publication of application: 31.05.2006
(73) Proprietor: AstraZeneca AB, 151 85 Södertälje (SE)
(72) Inventor: BAXTER, Andrew, AstraZeneca R & D Charnwood, Loughborough, Leicestershire LE11 5RH (GB); KINDON, Nicholas, AstraZeneca R & D Charnwood, Loughborough, Leicestershire LE11 5RH (GB); STOCKS, Michael, AstraZeneca R & D Charnwood, Loughborough, Leicestershire LE11 5RH (GB)
(86) International application number: PCT/SE2004/001227
(87) International publication number: WO 2005/021513

(56) References cited:
- EP-A1- 0 672 662
- EP-A2- 0 215 200
- WO-A1-97/32858
- WO-A1-03/051870
- WO-A1-03/059893
- DATABASE CAPLUS [Online] 2004:60488 'Preparation of pyrazine and quinoxaline derivatives as chemokine receptor CCR4 antagonists and medicinal use thereof', XP002903885 Retrieved from STN Database accession no. 2004:60488 & WO 2004 007472 A1

## Description

The present invention relates to a sulphonamide compound, processes and intermediates used in their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small-secreted molecules are a growing superfamily of 8-14 kDa proteins characterised by a conserved four cysteine motif. At the present time, the chemokine superfamily comprises three groups exhibiting characteristic structural motifs, the Cys-X-Cys (C-X-C), Cys-Cys (C-C) and Cys-X₃-Cys (C-X₃-C) families. The C-X-C and C-C families have sequence similarity and are distinguished from one another on the basis of a single amino acid insertion between the NH-proximal pair of cysteine residues. The C-X₃-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (M1P-1α and MIP-1β), Thymus and Activation Regulated Chemokine (TARC, CCL17) and Macrophage Derived Chemokine (MDC, CCL22). The C-X₃-C chemokine (also known as fractalkine) is a potent chemoattractant and activator of microglia in the central nervous system (CNS) as well as of monocytes, T cells, NK cells and mast cells.

Studies have demonstrated that the actions of chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family. These receptors represent good targets for drug development since agents which modulate these receptors would be useful in the treatment of disorders and diseases such as those mentioned above.

WO 03/059893 and WO 03/0511870 disclose a series of sulphonamide compounds said to be useful for treating various diseases.
EP1541563 (filing date 08.07.2003; publication date 15.06-2005) discloses compounds which have CCR4 antagonistic activity.
US patent 5,962,490 discloses a series of sulphonamide compounds said to be useful for treating endothelin mediated diseases. There is no specific disclosure of pyrazine or qunioxaline sulphonamides and no mention of chemokine mediated diseases.

The present invention therefore provides compounds of formulae (I) and pharmaceutically acceptable salts, solvates or N-oxides thereof: 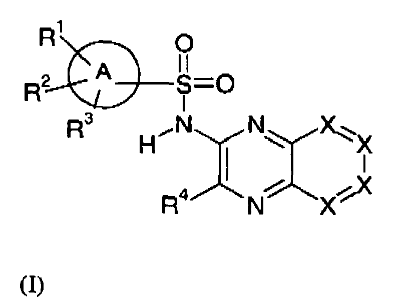 in which:
A is a thienyl, or phenyl ring;
X groups are independently N or CR⁵;
R¹ R² and R³ are independently hydrogen, halogen, cyano, CF₃, OCF₃, OC₁₋₆ alkyl or C₁₋₆ alkyl;
R⁴ is halogen,
C₁₋₆ alkoxy where the alkyl group may be substituted with 1-3 fluorine atoms or a cyano group,
C₃₋₆ alkenyloxy or C₃₋₆ alkenyloxy where either may be optionally substituted with hydroxy or NR⁶R⁷,
OC₁₋₆ alkylR⁸;
R⁵ is independently hydrogen, C₁₋₆ alkyl where the alkyl group may be substituted with 1-3 fluorine atoms, SO₂R⁶, SO₂NR⁶R⁷, cyano, halogen, CO₂R⁹, CONR⁶R⁷; (CH₂)ₙNR⁶R⁷, (CH₂)ₙOH, C₁₋₆ alkoxy where the alkyl group may be substituted with 1-3 fluorine atoms;
R⁶ and R⁷ are independently hydrogen, C₁₋₆ alkyl, C₃₋₆ cycloalkyl or (CH₂)_{q}OH,
or R⁶ and R⁷ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy,
R⁸ is an aryl group or a 5-7 membered heteraromatic ring containing 1-4 heteroatoms selected from nitrogen, oxygen or sulphur each of which can be optionally substituted by 1-3 groups selected from halogen, C(O)NR⁶R⁷, C(O)OR⁹, hydroxy, =O, =S, CN, NO₂, NR⁶R⁷, X(CH₂)_{q}NR⁶R⁷, (CH₂)ₙNR⁶R⁷, (CH₂)ₙOH;
R⁹ is independently hydrogen or C₁₋₆ alkyl where the alkyl group may be substituted with 1-3 fluorine atoms or may form a saturated 3-6 membered ring;
Y is O or S
n is 1 ,2, 3, 4 or 5; and
q is 2, 3, 4, 5 or 6, provided that the following compounds ar excluded:
N-(3-Methoxyquinoxalin-2-yl)thiophene-2-sulfonamide, and
N-[3-(2-Furylmethoxy)quinoxalin-2-yl]thiophene-2-sulfonamide
   and that when all X groups are independently CH, the unit AR¹R²R³ does not represent phenyl, 4-methylphenyl-, 4-fluorophenyl-, 4-chlorophenyl-, 4-ethylphenyl-, 4-propylphenyl-, 2-methylphenyl-, 3-methylphenyl-, 4-methoxypehyl-, 4-cyanophenyl-, 4-bromophenyl- or 4-(1-methylethyl)phenyl-.

The term aryl includes phenyl and naphthyl. The term alkyl, whether alone or as part of another group, includes straight chain and branched chain alkyl groups. Examples of 5- to 7-membered heteroaromatic ring containing 1 to 4 heteroatoms include thienyl, furanyl, pyrrolyl, imidazolyl, pyridyl, pyrazinyl, pyrimidyl, pyridazinyl, triazinyl, oxazolyl, thiazolyl, isoxazolyl, pyrazolyl, oxadiazolyl, thiadiazolyl, triazolyl and tetrazolyl.

Examples of saturated 4- to 8-membered rings containing 1 to 3 heteroatoms include morpholine, piperidine and azetidine. Substituents on any rings can be present in any suitable ring position including suitable substituents on nitrogen atoms.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

Preferably when A is thienyl, the thienyl moiety is linked to the sulphonamide at the 2-position of the thiophene ring. Preferably A is phenyl, optionally substituted as defined above.

When X groups are nitrogen, no more than two groups X can be nitrogen. Preferably all the groups X are CR⁵ or three are CR⁵ and the other is nitrogen. Preferably R⁵ is hydrogen, methoxy, chloro, or bromo. When R⁵ is methoxy, chloro, or bromo, it is preferred that one or two R⁵ groups are methoxy, chloro, or bromo and the other are hydrogen.

Preferred halogen groups for R¹, R² and R³ are chloro or bromo. Preferably R¹, R² and R³ are halogen or C₁₋₆ alkyl, in particular methyl. More preferably two of R¹, R² and R³ are chloro or one is methyl.

In an embodiment of the invention, R⁴ is halogen,
C₁₋₆ alkoxy where the alkyl group may be substituted with 1-3 fluorine atoms or a cyano group, or
C₃₋₆ alkenyloxy or C₃₋₆ alkynyloxy where either may be optionally substituted with hydroxy or NR⁶R⁷, with the provisas cited above.

In another embodiment of the invention, when R⁴ is OC₁₋₆ alkylR⁸ and each X group represents CH and A is phenyl and two of R¹R²R³ are hydrogen and the other is C₁₋₃ alkyl, methoxy, halogen or cyano excluding the cases cited above then R⁸ is not an unsubstituted phenyl, unsubstituted pyridyl, unsubstituted thienyl, unsubstituted pyrazolyl or 3,5-dimethyl-pyrazolyl group;
and when R⁴ is halogen, then the compound of formulae (I) is not N-(3-chloroquinoxalin-2-yl)-3-methyl-benzenesulfonamide.

Preferred groups for R⁴ include C₁₋₆ alkoxy such as methoxy and ethoxy. Most preferably R⁴ is methoxy.

Preferred compounds of formula (I) include:
2,3-Dichloro-N-(3-methoxyquinoxalin-2-yl)-benzenesulfonamide
5-Chloro-N-(3-methoxyquinoxalin-2-yl)thiophene-2-sulfonamide
2,3-Dichloro-N-(3,7-dimethoxyquinoxalin-2-yl)benzenesulfonamide
2,3-Dichloro-N-(2-methoxypyrido[2,3-b]pyrazin-3-yl)benzenesulfonamide
2,3-Dichloro-N-(3-methoxypyrido[2,3-b]pyrazin-2-yl)benzenesulfonamide
2,3-Dichloro-N-(3,6,7-trimethoxyquinoxalin-2-yl)benzenesulfonamide
2,3-Dichloro-N-(6,7-dichloro-3-methoxyquinoxalin-2-yl)benzenesulfonamide
*N*-(7-Bromo-3-methoxypyrido[2,3-*b*]pyrazin-2-yl)-2,3-dichlorobenzenesulfonamide
*N*-(7-Bromo-2-methoxypyrido[2,3-*b*]pyrazin-3-yl)-2,3-dichlorobenzenesulfonamide
2,3-Dichloro-*N*-(3-methoxypyrido[3,4-*b*]pyrazin-2-yl)benzenesulfonamide
and pharmaceutically acceptable salts and solvates thereof.

According to the invention there is also provided a process for the preparation of compounds of formula (I) which comprises reaction of a compound of formula (II): 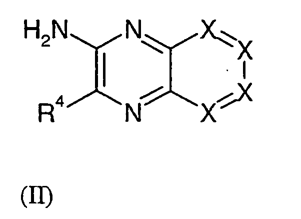 where R⁴ and X are as defined in formulae (I) or are protected derivatives thereof with a compound of formula (III): 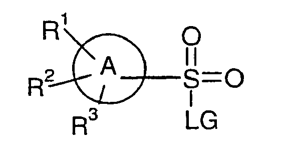 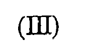 where R¹, R² and R³ are as defined in formulae (I) or are protected derivatives thereof and LG is a leaving group,
and optionally thereafter
• removing any protecting groups,
• converting a compound of formulae (I) to a further compound of formulae (I)
• forming a pharmaceutically acceptable salt.

Preferred leaving groups LG include halogen such as chloro. Preferably the reaction between compounds (II) with (III) is carried out by treating compound (II) with a base such as sodium hydride or potassium tert-butoxide in a suitable solvent such as 1,2-dimethoxyethane or tetrahydrofuran.

Compounds of formula (I) can also be prepared by treating a compound of the formulae (IV) where LG is a leaving group (such as chlorine or bromine): 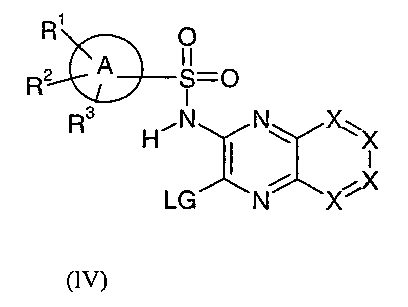 with a compound of formula (V)

HO-R¹⁰ (V)

where R¹⁰ can be C₁₋₆ alkoxy where the alkyl group may be substituted with 1-3 fluorine atoms or a cyano group;
C₃-₆ alkenyloxy or C₃₋₆ alkynyloxy where either may be optionally substituted with hydroxy or NR⁶R⁷;
OC₁₋₆ alkylR⁸, or OC₂₋₆ alkyl-Y-R⁸
where R⁶, R⁷ and R⁸ are as defined in formulae (I) and (II) in a suitable solvent (such as 1,2-dimethoxyethane, N,N-dimethylformamide or tetrahydrofuran) with a suitable base such as sodium hydride or potasssium tert-butoxide at a suitable temperature such as 25°C to 60°C.

According to the invention there is also provided a process for the preparation of compounds of formula (I) which comprises reaction of a compound of formula (VI): 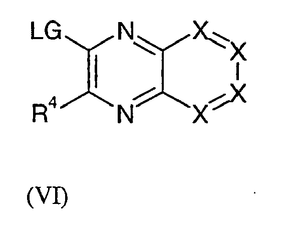 where R⁴ and X are as defined in formulae (I) or are protected derivatives thereof with a compound of formula (VII): 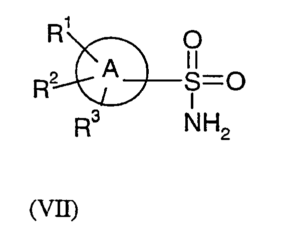 where R¹, R² and R³ are as defined in formulae (I) or are protected derivatives thereof and LG is a leaving group, ) in a suitable solvent (such as acetonitrile, or *N,N-*dimethylformamide) with a suitable base such as cesium carbonate or potasssium carbonate at a suitable temperature such as 25°C to 80°C. 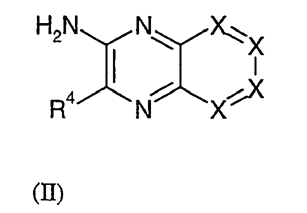 Compounds of formula (II) can be prepared by taking a compound of formula (VIII) 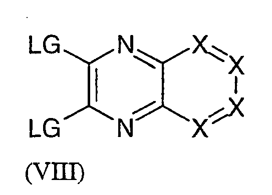 where LG is a suitable leaving group such as chlorine or bromine and reacting it with ammonia, generally concentrated aqueous solution, in a sealed reaction vessel at a temperature between 100-150°C, ideally between 110°C and 130°C to afford the compound (IX).

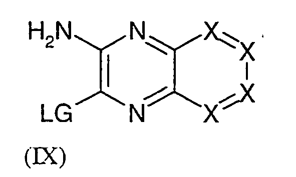

The resulting compound can then be treated with a compound of the formula (V), with a suitable base, such as sodium hydride in a suitable solvent, such as dimethoxyethane or N,N-dimethylformamide, at a temperature such as 20-70°C, ideally 50-60°C to afford compounds of the formula (II).

Compounds of formula (VIII) are either known in the literature or can be prepared from compounds of the formula (X) by reaction with a suitable chlorinating agent, such as phosphorus oxychloride, at a suitable temperature such as 100-120°C.

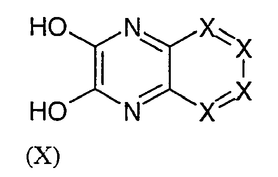

Compounds of formula (X) can be prepared by reacting a compound of formula (XI) with for example a compound such as diethyl oxalate at a temperature such as 150-180°C

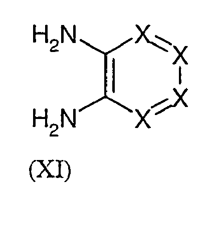

Compounds of the formula (III) may be prepared by standard literature methods or simple modifications of these methods by someone skilled in the art or are available commercially.

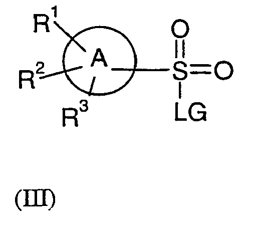

Compounds of formula (I) can also be prepared by taking a compound of formula (XII) where LG is as defined previously and reacting it with a compound of formula (VII) in a suitable solvent, such as N,N-dimethylformamide, 1-methyl-2-pyrrolidinone or acetonitrile with a suitable base, such as potassium carbonate or cesium carbonate at a suitable temperature, such as 50-100°C.

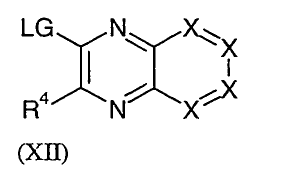

Compounds of formula (XII) can be prepared by taking a compound of formula (VIII) and reacting it with a compound of formula (V) in a suitable solvent, such as *N,N-*dimethylformamide, 1-methyl-2-pyrrolidinone, tetrahydrofuran or dimethoxyethane with asuitable base such as potassium tertbutoxide or sodium hydride at a suitable temperature, such as 50-100°C.

It will be appreciated by those skilled in the art that in the processes of the present invention certain functional groups such as hydroxyl or amino groups in the starting reagents or intermediate compound may need to be protected by protecting groups. Thus, the preparation of the compound of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley-Interscience (1991).

The compounds of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, meglumine tromethamine or procaine, or an acid addition salt such as a hydrochloride, hydrobromide, phosphate, acetate, fumarate, maleate, tartrate, citrate, oxalate, methanesulphonate or *p*-toluenesulphonate.

The compounds of formula (I) have activity as pharmaceuticals, in particular as modulators of chemokine receptor (especially CCR4) activity, and may be used in the treatment (therapeutic or prophylactic) of conditions/diseases in human and non-human animals which are exacerbated or caused by excessive or unregulated production of chemokines. Examples of such conditions/diseases include:
(1) (**the respiratory tract**) obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) (**bone and joints**) gout, rheumatoid arthritis, seronegative spondyloarthropathies (including ankylosing spondylitis, psoriatic arthritis and Reiter's disease), Behcet's disease, Sjogren's syndrome and systemic sclerosis;
(3) (skin) pruritis, scleroderma, otitus, psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis, lupus;
(4) (**gastrointestinal tract**) Coeliac disease, proctitis, eosinopilic gastro-enteritis, mastocytosis, inflammatory bowel diseases such as Crohn's disease, ulcerative colitis, ileitis and enteritis, food-related allergies which have effects remote from the gut, e.g., migraine, rhinitis and eczema;
(5) (**central and peripheral nervous system**) Neurodegenerative diseases and dementia disorders, e.g. Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia; polyneuropathies, e.g. Guillain-Barré syndrome, chronic inflammatory demyelinating polyradiculoneuropathy, multifocal motor neuropathy, plexopathies; CNS demyelination, e.g. multiple sclerosis, acute disseminated/haemorrhagic encephalomyelitis, and subacute sclerosing panencephalitis; neuromuscular disorders, e.g. myasthenia gravis and Lambert-Eaton syndrome; spinal diorders, e.g. tropical spastic paraparesis, and stiff-man syndrome: paraneoplastic syndromes, e.g. cerebellar degeneration and encephalomyelitis; CNS trauma; migraine; stroke and correctum diseases such as meningitis
(6) (**other tissues and systemic disease**) hepatitis, vasculitis, spondyloarthopathies, vaginitis, glomerulonephritis, myositis, atherosclerosis, Acquired Immunodeficiency Syndrome (AIDS), lupus erythematosus, systemic lupus, erythematosus, Hashimoto's thyroiditis, type I diabetes, nephrotic syndrome, eosinophilia fascitis, hyper IgE syndrome, lepromatous leprosy, and idiopathic thrombocytopenia pupura; post-operative adhesions, and sepsis.
(7) (allograft and xenograft rejection) acute and chronic following, for example, transplantation of kidney, heart, liver, lung, bone marrow, skin and cornea; and chronic graft versus host disease;
(8) Cancer, carcinoma & tumour metastasis, including that of the bladder, breast, colon, kidney, liver, lung, ovary, pancreas, stomach, cervix, thyroid and skin, especially non-small cell lung cancer (NSCLC), malignant melanoma, prostate cancer and squamous sarcoma. Hematopoietic tumors of lymphoid lineage, including acute lymphocytic leukemia, B cell lymphoma and Burketts lymphoma, Hodgkins Lymphoma, Acute Lymphoblastic Leukemia. Hematopoietic tumors of myeloid lineage, including acute and chronic myelogenous leukemias and promyelocytic leukemia. Tumors of mesenchymal origin, including fibrosarcoma and rhabdomyosarcoma, and other tumors, including melanoma, seminoma, tetratocarcinoma, neuroblastoma and glioma.
(9) All diseases that result from a general inbalance of the immune system and resulting in increased atopic inflammatory reactions.
(10)Cystic fibrosis, re-perfusion injury in the heart, brain, peripheral limbs and other organs.
(11) Bum wounds & chronic skin ulcers
(12) Reproductive Diseases (e.g. Disorders of ovulation, menstruation and implantation, Pre-term labour, Endometriosis)
(13) thrombosis
(14) infectious diseases such as HIV infection and other viral infections, bacterial infections.

Thus, the present invention provides a compound of formula (I), or a pharmaceutically-acceptable salt or solvate thereof, as hereinbefore defined for use in therapy.

Preferably the compound of the invention are used to treat diseases in which the chemokine receptor belongs to the CC chemokine receptor subfamily, more preferably the target chemokine receptor is the CCR4 receptor.

Particular conditions which can be treated with the compound of the invention are asthma, rhinitis and inflammatory skin disorders, diseases in which there are raised TARC, MDC or CCR4 levels. It is preferred that the compound of the invention is used to treat asthma and rhinitis, especially asthma.

In a further aspect, the present invention provides the use of a compound of formula (I), or a pharmaceutically acceptable salt or solvate thereof, as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity, particularly CCR4 activity, is beneficial.

In the context of the present specification, the term "therapy" also includes "prophylaxis" unless there are specific indications to the contrary. The terms "therapeutic" and "therapeutically" should be construed accordingly.

The invention still further provides the use of a compound of formula (IA), or a pharmaceutically acceptable salt or solvate thereof, in the manufacture of a medicament for treating a chemokine mediated disease. 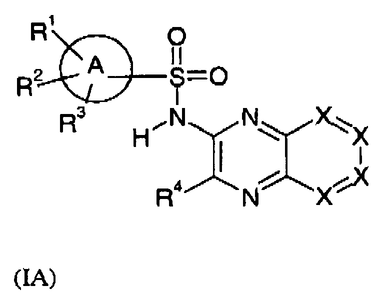 in which:
A is a thienyl or phenyl ring;
X groups are independently N or CR⁵;
R¹, R² and R³ are independently hydrogen, halogen, cyano, CF₃, OCF₃, OC₁₋₆ alkyl or C₁₋₆ alkyl;
R⁴ is halogen,
C₁₋₆ alkoxy where the alkyl group may be substituted with 1-3 fluorine atoms or a cyano group,
C₃₋₆ alkenyloxy or C₃₋₆ alkynyloxy where either may be optionally substituted with hydroxy or NR⁶R⁷,
OC₁₋₆ alkylR⁸;
R⁵ is independently hydrogen, C₁₋₆ alkyl where the alkyl group may be substituted with 1-3 fluorine atoms, SO₂R⁶, SO₂NR⁶R⁷, cyano, halogen, CO₂R⁹, CONR⁶R⁷; (CH₂)ₙNR⁶R⁷, (CH₂)ₙOH, C₁₋₆ alkoxy where the alkyl group may be substituted with 1-3 fluorine atoms;
R⁶ and R⁷ are independently hydrogen, C₁₋₆ alkyl, C₃₋₆ cycloalkyl or (CH₂)_{q}OH,
or R⁶ and R⁷ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy,
R⁸ is an aryl group or a 5-7 membered heteraromatic ring containing 1-4 heteroatoms selected from nitrogen, oxygen or sulphur each of which can be optionally substituted by 1-3 groups selected from halogen, C(O)NR⁶R⁷, C(O)OR⁹, hydroxy, =O, =S, CN, NO₂, NR⁶R⁷, X(CH₂)_{q}NR⁶R⁷, (CH₂)ₙNR⁶R⁷, (CH₂)ₙOH;
R⁹ is independently hydrogen or C₁₋₆ alkyl where the alkyl group may be substituted with 1-3 fluorine atoms or may form a saturated 3-6 membered ring;
Y is O or S
n is 1 ,2, 3, 4 or 5; and
q is 2, 3, 4, 5 or 6.
   provided that when all X groups are independently CH, the unit AR¹R²R³ does not represent phenyl, 4-methylphenyl-, 4-fluorophenyl-, 4-chlorophenyl-, 4-ethylphenyl-, 4-propylphenyl-, 2-methylphenyl-, 3-methylphenyl-, 4-methoxyphenyl-, 4-cyanophenyl-, 4-bromophenyl- or 4-(1-methylethyl)phenyl-.

The invention also provides a method of treating a respiratory disease, such as asthma and rhinitis, especially asthma, in a patient suffering from, or at risk of, said disease, which comprises administering to the patient a therapeutically effective amount of a compound of formula (IA), or a pharmaceutically acceptable salt or solvate thereof, as hereinbefore defined.

For the above-mentioned therapeutic uses the dosage administered will, of course, vary with the compound employed, the mode of administration, the treatment desired and the disorder indicated.

The compound of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention further relates to combination therapies for the treatment of any one of rheumatoid arthritis, osteoarthritis, osteoporosis, psoriasis, inflammatory bowel diseases, COPD, asthma, allergic rhinitis, atopic dermatitis or cancer or the neurodegenerative diseases such as multiple sclerosis, Alzheimer's disease or stroke.

For the treatment of rheumatoid arthritis, the compounds of the invention may be combined with "biological agents" such as TNF-α inhibitors such as anti-TNF monoclonal antibodies (such as Remicade, CDP-870 and Humira) and soluble TNF receptor immunoglobulin molecules (such as Enbrel.reg.). IL-1 receptor antagonist (such as Anakinra) and IL-1 trap, IL-18 receptor, anti-IL-6 Ab, anti-CD20 Ab, anti-IL-15 Ab and CTLA4Ig.

Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin. The cyclooxygenase-2 (COX-2) inhibitors (such as meloxicam, celecoxib , rofecoxib, valdecoxib and etoricoxib) and the cyclo-oxygenase inhibiting nitric oxide donors (CINOD's) and the "disease modifying agents" (DMARDs) such as methotrexate, sulphasalazine, cyclosporine A, lefunomide; ciclesonide; hydroxychloroquine, d-penicillamine, auranofin or parenteral or oral gold.

The present invention still further relates to the combination of a compound of the invention together with a leukotriene biosynthesis inhibitor, 5-lipoxygenase (5-LO) inhibitor or 5-lipoxygenase activating protein (FLAP) antagonist selected from the group consisting of zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; the compound SB-210661; pyridinyl-substituted 2n cyanonaphthalene compounds such as L-739,010; 2-cyanoquinoline compounds such as L-746,530; indole and quinoline compounds such as MK-591, MK-886, and BAY x 1005.

The present invention still further relates to the combination of a compound of the invention together with a receptor antagonists for leukotrienes LTB₄, LTC₄, LTD₄, and LTE₄ selected from the group consisting of the phenothiazin-3-ones such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a phosphodiesterase-4 (PDE4) inhibitor including inhibitors of the isoform PDE4D.

The present invention still further relates to the combination of a compound of the invention together with histaminic H₁ receptor antagonists including cetirizine, loratadine, desloratadine, fexofenadine, astemizole, azelastine, and chlorpheniramine.

The present invention still further relates to the combination of a compound of the invention together with a gastroprotective histaminic H₂ receptor antagonist or the proton pump inhibitors (such as omeprazole)

The present invention still further relates to the combination of a compound of the invention together with an α₁- and α₂-adrenoceptor agonist vasoconstrictor sympathomimetic agent, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents including ipratropium bromide; tiotropium bromide; oxitropium bromide; pirenzepine; and telenzepine.

The present invention still further relates to the combination of a compound of the invention together with a β₁- to β₄-adrenoceptor agonists including metaproterenol isoproterenol, isoprenaline, albuterol, salbutamol, formoterol, salmeterol, terbutaline, orciprenaline, bitolterol mesylate, and pirbuterol; or methylxanthanines including theophylline and aminophylline; sodium cromoglycate; or muscarinic receptor (M1, M2, and M3) antagonist.

The present invention still further relates to the combination of a compound of the invention together with other modulators of chemokine receptor function such as CCR1, CCR2, CCR2A, CCR2B, CCR3, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention still further relates to the combination of a compound of the invention together with an insulin-like growth factor type I (IGF-1) mimetic.

The present invention still further relates to the combination of compound of the invention together with an inhaled glucocorticoid with reduced systemic side effects, including prednisone, prednisolone, flunisolide, triamcinolone acetonide, beclomethasone dipropionate, budesonide, fluticasone propionate, and mometasone furoate.

The present invention still further relates to the combination of a compound of the invention together with (a) tryptase inhibitors; (b) platelet activating factor (PAF) antagonists; (c) interleukin converting enzyme (ICE) inhibitors; (d) IMPDH inhibitors; (e) adhesion molecule inhibitors including VLA-4 antagonists; (f) cathepsins; (g) MAP kinase inhibitors; (h) glucose-6 phosphate dehydrogenase inhibitors; (i) kinin-B₁ - and B₂ - receptor antagonists; (j) anti-gout agents, e.g., colchicine; (k) xanthine oxidase inhibitors, e.g., allopurinol; (I) uricosuric agents, e.g., probenecid, sulfinpyrazone, and benzbromarone; (m) growth hormone secretagogues; (n) transforming growth factor (TGFβ); (o) platelet-derived growth factor (PDGF); (p) fibroblast growth factor, e.g., basic fibroblast growth factor (bFGF); (q) granulocyte macrophage colony stimulating factor (GM-CSF); (r) capsaicin cream; (s) Tachykinin NK₁ and NK₃ receptor antagonists selected from the group consisting of NKP-608C; SB-233412 (talnetant); and D-4418; and (t) elastase inhibitors selected from the group consisting of UT-77 and ZD-0892 (u) induced nitric oxide synthase inhibitors (iNOS) or (v) chemoattractant receptor-homologous molecule expressed on TH2 cells, (CRTH2 antagonists).

The present invention still further relates to the combination of a compound of the invention together with an inhibitor of matrix metalloproteases (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), stromelysin-3 (MMP-11), and MMP12 inhibitors.

The compounds of the invention can also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, induced nitric oxide synthase inhibitors (iNOS inhibitors), COX-2 inhibitors such as celecoxib, valdecoxib, rofecoxib and etoricoxib, and the cyclo-oxygenase inhibiting nitric oxide donors (CINOD's) analgesics (such as paracetamol and tramadol), cartilage sparing agents such as diacerein, doxycyline and glucosamine, and intra-articular therapies such as corticosteroids and hyaluronic acids such as hyalgan and synvisc and P2X7 antagonists.

The compounds of the invention can also be used in combination with existing therapeutic agents for the treatment of inflammatory bowel diseases (Ulcerative colitis and Crohn's disease). Suitable agents to be used include sulphasalazine, 5-amino-salicylates, the thiopurines, azathioprine and 6-mecaptorurine and corticosteroids such as budesonide.

The compounds of the invention may also be used in combination with antiviral agents such as Viracept, AZT, aciclovir and famciclovir, and antisepsis compounds such as Valant.

The compounds of the present invention may also be used in combination with cardiovascular agents such as calcium channel blockers, lipid lowering agents such as statins, fibrates, beta-blockers, angiotensin converting enzyme (ACE) inhibitors, Angiotensin-2 receptor antagonists and platelet aggregation inhibitors.

The compounds of the present invention may also be used in combination with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti Alzheimer's drugs such as donepezil, tacrine, COX-2 inhibitors, propentofylline or metryfonate.

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside, hydroxyurea, gemcitabine and paclitaxel (Taxol®); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5a-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]) , farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO99/02166, WO00/40529, WO00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The following examples illustrate the invention.

### Example 1

### 2,3-Dichloro-N-(3-methoxyquinoxalin-2-yl)-benzenesulfonamide

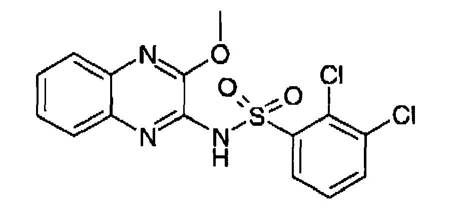

Sodium hydride (0.07g of 60% w/w in oil) was added to 3-methoxy-2-quinoxalinamine (0.15g) in 1,2-dimethoxyethane (5mL) under nitrogen at room temperature. After 30 minutes, 2,3-dichlorobenzenesufonyl chloride (0.204g) was added. After 3 hours further sodium hydride (0.03g of 60% w/w in oil) and 2,3-dichlorobenzenesufonyl chloride (0.03g) were added. After stirring for 16 hours, 5% aqueous citric acid was added and the product extracted with ethyl acetate (3 x 20mL). The combined extracts were washed with brine, dried over anhydrous magnesium sulfate and the solvent was evaporated. Chromatography on silica gel eluting with *iso*hexane/ethyl acetate/methanol mixtures gave the title compound as a white solid (0.033g).
m/e 382/4/6 (M-1⁺)
¹H NMR (D6-DMSO) δ 8.31 (1H, s); 7.94 (1H, d); 7.73-7.61 (3H, m); 7.54-7.44 (3H, m); 4.03 (3H, s).

### Example 2 (not part of the present invention)

### N-(3-Methoxyquinoxalin-2-yl)-4-methylbenzenesulfonamide

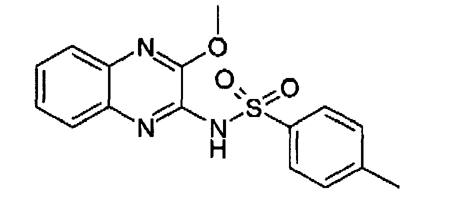

Obtained as a commercial sample from Labotest.

### Example 3

### 5-Chloro-N-(3-methoxyquinoxalin-2-yl)thiophene-2-sulfonamide

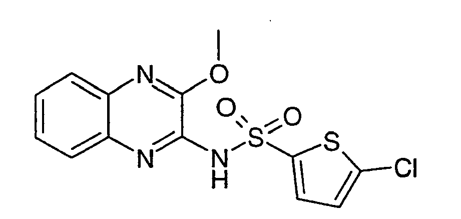

Sodium hydride (0.07g of 60% w/w in oil) was added to 3-methoxy-2-quinoxalinamine (0.15g) in 1,2-dimethoxyethane (5mL) under nitrogen at room temperature. After 30 minutes, 5-chloro-2-thienylsufonyl chloride (0.184g) was added. After 3 hours further sodium hydride (0.04g of 60% w/w in oil) and 5-chloro-2-thienylsufonyl chloride (0.04g) were added. After stirring for 16 hours, 5% aqueous citric acid was added and the product extracted with ethyl acetate (3x20mL). The combined extracts were washed with brine, dried (MgSO₄) and the solvent was evaporated. Chromatography on silica gel eluting with isohexane/ethyl acetate/methanol mixtures gave the title compound as a white solid (0.35g).
m/e 356/8 (M-1⁺)
¹H NMR (D6-DMSO) δ 7.93-7.87 (1H, m); 7.82 (1H, d); 7.78-7.69 (1H, m); 7.62-7.53 (2H, m); 7.25 (1H, d); 4.04 (3H, s).

### Example 4

### N-(3-Methoxyquinoxalin-2-yl)thiophene-2-sulfonamide

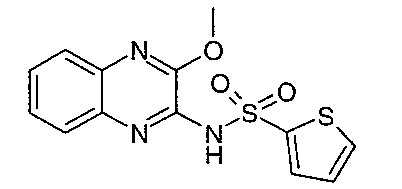

Obtained as a commercial sample from Enamine, Kiev, Ukraine

### Example 5

### N-[3-(2-Furylmethoxy)quinoxalin-2-yl]thiophene-2-sulfonamide

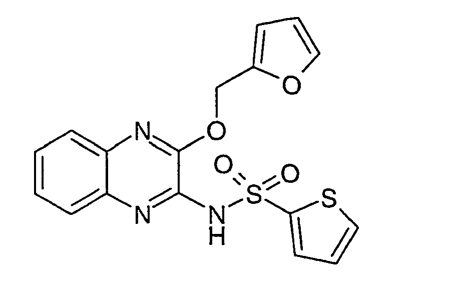

Obtained as a commercial sample from Enamine, Kiev, Ukraine

### Example 6

### 2,3-Dichloro-N-(3,7-dimethoxyquinoxalin-2-yl)benzenesulfonamide

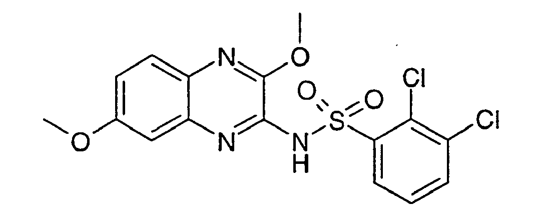

a) 2,3-Dichloro-N-(3-chloro-7-methoxyquinoxalin-2-yl)benzenesulfonamide
   A mixture of 2,3-dichlorobenzenesulfonamide (0.5g) and cesium carbonate (0.72g) was stirred together for 10 minutes in 1-methyl-2-pyrrolidinone (10mL) at rt. Then 2,3-dichloro-6-methoxyquinoxaline (0.51g) was added and the resulting mixture heated to 100°C for 24 hours. The reaction mixture was cooled and then acidified to pH 3 using 2M hydrocloric acid. The product was extracted with ethyl acetate (3x20mL). The combined extracts were dried over anyhdrous magnesium sulfate, filtered and concentrated to give the sub-titled compound as a yellow solid (0.62g).
b) To a suspension of 2,3-dichloro-N-(3-chloro-7-methoxyquinoxalin-2-yl)benzenesulfonamide (Example 6a, 0.62g) in methanol was added a solution of 3N sodium methoxide in methanol (4.7mL). The reaction mixture was heated to reflux for 24 hours. The reaction mixture was cooled and then acidified to pH6 using 2M hydrochloric acid. The product was extracted with ethyl acetate (3x20mL). The combined extracts were dried over anhydrous magnesium sulfate, filtered and then concentrated. The residue was purified by chromatography on silica gel eluting with isohexane:ethyl acetate 9:1 to 8:2 to give the title compound as a white solid (0.163g).
   m/e 412 (M+1⁺)
   ¹H NMR (D6-DMSO, main regioisomer) δ 8.27 (1H, br s); 7.99-7.89 (1H, m); 7.64-7.54 (2H, m); 7.15-7.10 (1H, m); 6.86 (1H, br s); 4.02(3H, s); 3.83 (3H, s).

### Examples 7 and 8

### 2,3-Dichloro-N-(2-methoxypyrido[2,3-b]pyrazin-3-yl)benzenesulfonamide and 2,3-dichloro-N-(3-methoxypyrido[2,3-b]pyrazin-2-yl)benzenesulfonamide

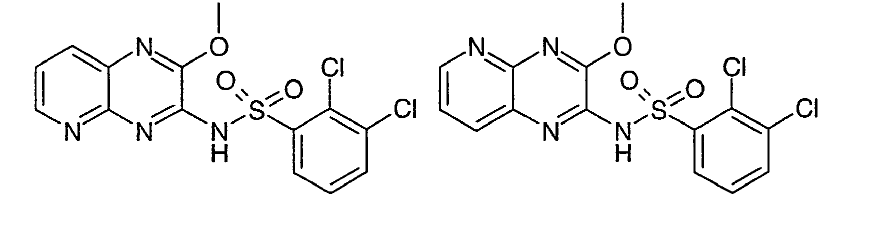

a) 2-Chloro-3-methoxy-pyrido[2,3-b]pyrazine
   To a solution of 2,3-dichloro-pyrido[2,3-b]pyrazine (0.5g) in methanol was added 0.3N solution of sodium methoxide (6.5ml). The resulting mixture was heated to reflux for 4 hours allowed to cool and used in example (7b).
b) A mixture of 2,3-dichloro-benzenesulfonamide (0.59g) and cesium carbonate (0.85g) were stirred together for 10 minutes in 1-methyl-2-pyrrolidinone (10mL) at rt. Then the reaction mixture from Example 7a was added dropwise and the resulting mixture heated to 120°C for 24 hours. The reaction mixture was cooled, adsorbed on to silica gel and purified by chromatography on silica gel eluting with dichloromethane:methanol 98:2 to 95:5.The residue (0.062g) obtained was further purified by RPHPLC using 0.2% aqueous TFA / acetonitrile 85:35. This gave the titled compounds as (TFA salts) in order of elution:
   2,3-Dichloro-N-(2-methoxypyrido[2,3-b]pyrazin-3-yl)benzenesulfonamide as a yellow solid (0.017g).
      m/e 385 (M+1⁺)
      ¹H NMR (D6-DMSO) δ 8.36-8.34 (2H, m); 8.19 (1H, d); 7.80 (1H, d); 7.53-7.45 (2H, m); 4.00 (3H, s). M.P. 238-239°C
   2,3-Dichloro-N-(3-methoxypyrido[2,3-b]pyrazin-2-yl) as a white solid (0.012g).
      m/e 385 (M+1⁺)
      ¹H NMR (D6-DMSO) δ 8.40 ( 1H, br d); 8.29 (1H, br s); 7.93 (1H, d); 7.62 (1H, t); 7.29 (1H, dd); 3.63 (3H, s).
      M.P. 217-218°C

### Example 9

### 2,3-Dichloro-N-(3,6,7-trimethoxyquinoxalin-2-yl)benzenesulfonamide

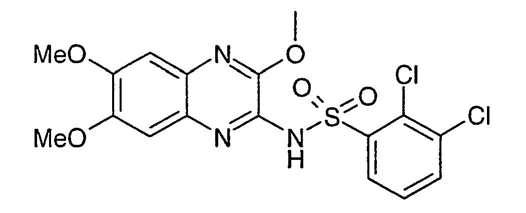

a) 2,3-Dichloro-N-(3-chloro-6,7-dimethoxyquinoxalin-2-yl)benzenesulfonamide
   A mixture of 2,3-dichlorobenzenesulfonamide (0.255g) and cesium carbonate (0.368g) and 2,3-dichloro-6,7-dimethoxyquinoxaline (0.292g) were heated together in 1-methyl-2-pyrrolidinone (10mL) at 100°C for 24 hours. The reaction mixture was allowed to cool and used in example 9b. m/e 448 (M+1⁺)
b) To the reaction mixture from example (9a) was added methanol (10mL) and then 3N sodium methoxide solution in methanol (3.6ml). The reaction mixture was heated to reflux for 24 hours and then allowed to cool. The reaction mixture was poured onto water and acidified to pH 3/4 with 2M hydrochloric acid. The product was extracted into ethyl acetate (3x20mL) and the combined organic extracts were washed with water (7x10mL), dried over anhydrous magnesium sulfate and filtered. The filtrate was adsorbed onto silica gel and purified by chromatography on silica geleluting with isohexane:ethyl acetate 8:2, 1:1, methanol. The residue obtained (0.1g) was triturated in methanol and collected by filtration. This was further purified by RPHPLC using 0.2% aqueous ammonium acetate / acetonitrile 95:05. This gave the title compound as a beige solid (0.0189g)
   m/e 442 (M-1⁺)
   ¹H NMR (D6-DMSO) δ 8.28 (1H, broad s); 7.93 (1H, d); 7.64 (1H, t); 7.14 (1H, s); 6.84 (1H, broad s); 3.98 (3H, s); 3.84 (6H, d).
   M.P. 215°C.

### Example 10

### 2,3-Dichloro-N-(6,7-dichloro-3-methoxyquinoxalin-2-yl)benzenesulfonamide

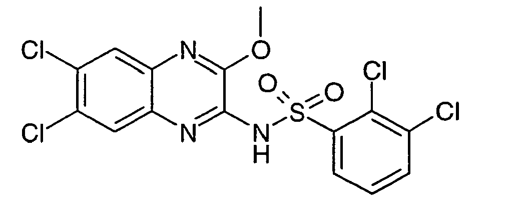

a) 2,3-Dichloro-N-(3,6,7-trichloroquinoxalin-2-yl)benzenesulfonamide
   A mixture of 2,3-dichlorobenzenesulfonamide (0.21g) and cesium carbonate (0.30g) were stirred together for 10 minutes in 1-methyl-2-pyrrolidinone (10mL) at rt. Then 2,3,6,7-tetrachloroquinoxaline (0.25g) was added and the resulting mixture heated to 70°C for 24 hours. The reaction mixture was cooled, poured onto water and then acidified to pH 3/4 using 2M hydrochloric acid. The product was extracted with ethyl acetate (3x30mL). The combined extracts were washed with water (6x10mL), dried over anyhdrous magnesium sulfate, filtered and concentrated to give the sub-titled compound as a yellow solid (0.35g). m/e 458 (M+1⁺).
b) Sodium methoxide (1.155mmol) in methanol (3.68ml) was added to 2,3-dichloro-N-(3,6,7-trichloroquinoxalin-2-yl)benzenesulfonamide (Example 10a, 0.35g) in methanol (10mL). The resulting mixture was heated to reflux for 24 hours. Then the cooled reaction mixture was adsorbed onto silica gel and purified by chromatography on silica gel eluting with isohexane: ethyl acetate 8:2, 1:1 to give the title compound as a yellow solid (0.102g).
   M/e 454 (M+1⁺, 98.8%)
   ¹H NMR (D6-DMSO) δ 8.26 (1H, d);7.81-7.77 (2H, m); 7.57 (1H, t); 7.39 (1H, br s); 3.97 (3H, s).
   M.P. 283-285°C.

### Example 11 and 12

### N-(7-Bromo-3-methoxypyrido[2,3-b]pyrazin-2-yl)-2,3-dichlorobenzenesulfonamide and N-(7-bromo-2-methoxypyrido[2,3-b]pyrazin-3-yl)-2,3-dichlorobenzenesulfonamide

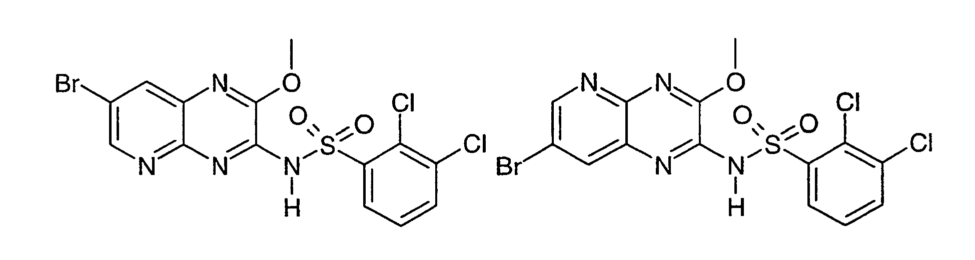

A stirred mixture of 7-bromo-2,3-dichloropyrido[2,3-*b*]pyrazine (3.2g), cesium carbonate (3.91g) and 2,3-dichlorobenzenesulfonamide (2.71g) were mixed together in dry acetonitrile (100mL) and heated to 60°C for 5 hours. The reaction mixture was cooled and concentrated. The residue was suspended in methanol (50mL) and a solution of sodium methoxide in methanol (25 wt%, 10mL) was added and the resulting suspension stirred at 60°C for 3 hours. The reaction mixture was cooled and concentrated and the residue partitioned between ethyl acetate (100mL) and water (100mL). The aqueous layer was acidified with saturated citric acid and the organic layer extracted into ethyl acetate (2x100mL). The combined extracts were dried over anhydrous magnesium sulfate, filtered and concentrated. The residue was purified by chromatography on silical gel eluting with 1-5% methanol in dichloromethane to give the titled compounds in order of elution:
*N*-(7-bromo-3-methoxypyrido[2,3-*b*]pyrazin-2-yl)-2,3-dichlorobenzenesulfonamide m/e 462/464 (M+1⁺)
   ¹H NMR (DMSO) δ 8.68 (1H, d); 8.40(1H, d); 8.23 (1H, dd); 7.91 (1H, dd); 7.62 (1H, t); 4.06 (3H, s).
   M.P. 230-231°C
*N*-(7-bromo-2-methoxypyrido[2,3-*b*]pyrazin-3-yl)-2,3-dichlorobenzenesulfonamide
   m/e 463/465 (M-1⁺)
   ¹H NMR (DMSO) δ 8.74 (1H, d); 8. 34 (1H, dd); 8.20 (1H, br s); 7.93 (1H, dd); 7.65 (1H, t); 4.08 (3H, s).
   M.P. 233-234°C

### Example 13

### 2,3-Dichloro-N-(3-methoxypyrido[3,4-b]pyrazin-2-yl)benzenesulfonamide

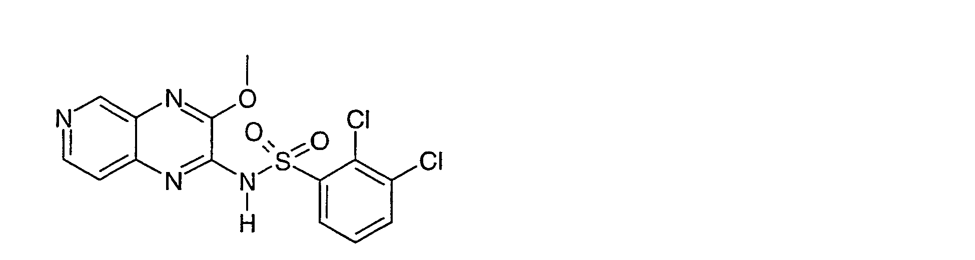

A suspension of 2,3-dichloropyrido[3,4-*b*]pyrazine (0.15g), cesium carbonate (0.326g) and 2,3-dichlorobenzenesulfonamide (0.181g) in dry acetonitrile (10mL) was stirred at room temperature for 20 hours. The reaction mixture was concentrated and the solvent was exchanged for dry methanol (10mL) and a solution of sodium methoxide in methanol (25 wt%, 2mL) was added and the resulting suspension stirred at 60°C for 3 hours. The reaction mixture was cooled and concentrated and the residue partitioned between ethyl acetate (100mL) and water (100mL). The aqueous layer was acidified with saturated citric acid and the organic layer extracted into ethyl acetate (2x100mL). The combined extracts were dried over anhydrous magnesium sulfate, filtered and concentrated. The residue was purified by chromatography on silical gel eluting with 10% methanol in dichloromethane to give the titled compound as an off-white solid (0.030g):
m/e 385/387 (M+1⁺)
¹H NMR (DMSO) δ 8.91 (1H, s); 8.27(1H, d); 8.19 (1H, d); 7.76 (1H, d); 7.53 (1H, t); 7.16 (1H, t); 4.00 (3H, s).
M.P. 256-257°C.

### Pharmacological Data

### FMAT Whole cell binding assay

### Cells

CHO-K1 cells stably expressing the human recombinant CCR4 receptor (Euroscreen; Brussels, Belgium) were cultured in NUT.MIX.F_12(HAM) medium with glutamax-1, containing 10% (v/v) foetal bovine serum and 400 µg ml⁻¹ geneticin.

Cells were harvested at approximately 70% confluence by treatment with a cell dissociation buffer, and seeded at 5x10³ cells/100µl culture medium into wells of a black Costar clear-bottomed 96-well microtitre plates. Plates were incubated overnight at 37°C in 5% CO₂ and used the following day.

### ASSAY

Before use, the cell plates were washed twice with 100 µl Hanks balanced salt solution (HBSS). To each well was then added 65µl of HBSS, 10 µL of 10% DMSO in HBSS ± test compound and then 25 µl of 2.8 nM FB-MDC (Applied Biosystems). This fluorescent probe was prepared from a 10 µM stock in 0.08% (v/v) TFA/16% (v/v) acetonitrile, diluted into HBSS.

After two hours incubation in the dark at room temperature, the plates were analysed in an FMAT8100 reader (Applied Biosystems) to measure fluorescence that was associated with binding of FB-MDC to the cells. Compound activity was determined as an pIC₅₀ [log(concentration of compound that results in 50% inhibition)], comparing fluorescence in control and background wells.

### Typical Data

Fluorescence (ctrl) = 1200
Fluorescence (bkg) = 0

| | | Mean |
|---|---|---|
| Example 1 | pIC₅₀ | 5.8 |
| Example 3 | pIC₅₀ | 6.4 |
| Example 5 | pIC₅₀ | 5.7 |

All the compounds of the examples have a pIC₅₀ of greater than 5.0.

## Claims

1. A compound of formula (I) and pharmaceutically acceptable salts or solvates thereof: 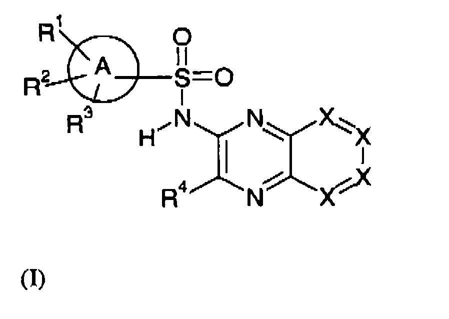 in which:
A is a thienyl or phenyl, ring;
X groups are independently N or CR⁵;
R¹, R² and R³ are independently hydrogen, halogen, cyano, CF₃, OCF₃, OC₁₋₆ alkyl or C₁₋₆ alkyl;
R⁴ is halogen,
C₁₋₆ alkoxy where the alkyl group may be substituted with 1-3 fluorine atoms or a cyano group,
C₃₋₆ alkenyloxy or C₃₋₆ alkynyloxy where either may be optionally substituted with hydroxy or NR⁶R⁷,
OC₁₋₆ alkylR⁸;
R⁵ is independently hydrogen, C₁₋₆ alkyl where the alkyl group may be substituted with 1-3 fluorine atoms, SO₂R⁶, SO₂NR⁶R⁷, cyano, halogen, CO₂R⁹, CONR⁶R⁷; (CH₂)ₙNR⁶R⁷ (CH₂)ₙOH, C₁₋₆ alkoxy where the alkyl group may be substituted with 1-3 fluorine atoms;
R⁶ and R⁷ are independently hydrogen, C₁₋₆ alkyl, C₃₋₆ cycloalkyl or (CH₂)_{q}OH,
or R⁶ and R⁷ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy,
R⁸ is an aryl group or a 5-7 membered heteraromatic ring containing 1-4 heteroatoms selected from nitrogen, oxygen or sulphur each of which can be optionally substituted by 1-3 groups selected from halogen, C(O)NR⁶R⁷, C(O)OR⁹, hydroxy, =O, =S, CN, NO₂ NR⁶R⁷, X(CH₂)_{q}NR⁶R⁷, (CH₂)ₙNR⁶R⁷, (CH₂)ₙOH;
R⁹ is independently hydrogen or C₁₋₆ alkyl where the alkyl group may be substituted with 1-3 fluorine atoms or may form a saturated 3-6 membered ring;
Y is O or 5
n is 1 ,2, 3, 4 or 5; and
q is 2, 3, 4, 5 or 6, provided that the following compounds are excluded:
N-(3-Methoxyquinoxalin-2-yl)thiophene-2-sulfonamide, and
N-[3-(2-Furylmethoxy)quinoxalin-2-yl]thiophene-2-sulfonamide
and that when all X groups are independently CH, the unit AR¹R²R³ does not represent phenyl, 4-methylphenyl-, 4-fluorophenyl-, 4-chlorophenyl-, 4-ethylphenyl-, 4-propylphenyl-, 2-methylphenyl-, 3-methylphenyl-, 4-methoxypehyl-, 4-cyanophenyl-, 4-bromophenyl- or 4-(1-methylethyl)phenyl-.

2. A compound according to claim 1 in which A is phenyl.

3. A compound according to claim 1 or 2 in which two of R¹, R² and R³ are chloro or one is methyl.

4. A compound according to any one of claims 1 to 3 in which R⁴ is
C₁₋₆ alkoxy where the alkyl group may be substituted with 1-3 fluorine atoms or a cyano group, or
C₁₋₆ alkenyloxy or C₃₋₆ alkynyloxy where either may be optionally substituted with hydroxy or NR⁶R⁷.

5. A compound according to any one of claims 1 to 4 in which R⁴ is C₁₋₆ alkoxy.

6. A compound according to claim 1 which is:
2,3-Dichloro-N-(3-methoxyquinoxalin-2-yl)-benzenesulfonamide
5-Chloro-N-(3-methoxyquinoxalin-2-yl)thiophene-2-sulfonamide
2,3-Dichloro-N-(3,7-dimethoxyquinoxalin-2-yl)benzenesulfonamide
2,3-Dichloro-N-(2-methoxypyrido[2,3-b]pyrazin-3-yl)benzenesulfonamide
2,3-Dichloro-N-(3-methoxypyrido[2,3-b]pyrazin-2-yl)benzenesulfonamide
2,3-Dichloro-N-(3,6,7-trimethoxyquinoxalin-2-yl)benzenesulfonamide
2,3-Dichloro-N-(6,7-dichloro-3-methoxyquinoxalin-2-yl)benzenesulfonamide
*N*-(7-Bromo-3-methoxypyrido[2,3-*b*]pyrazin-2-yl)-2,3-dichlorabenzenesulfonamide
*N*-(7-Bromo-2-methoxypyrido[2,3-*b*]pyrazin-3-yl)-2,3-dichlorobenzenesulfonamide
2,3-Dichloro-*N*-(3-methoxypyrido[3,4-*b*]pyrazin-2-yl)benzenesulfonamide
and pharmaceutically acceptable salts and solvates thereof.

7. A pharmaceutical composition comprising a compound of formula (I), or a pharmaceutically acceptable salt or solvate thereof, as claimed in claims 1 to 6 in association with a pharmaceutically acceptable adjuvant, diluent or carrier.

8. A process for the preparation of a pharmaceutical composition as claimed in claim 2 which comprises mixing a compound of formula (I), or a pharmaceutically acceptable salt or solvate thereof, as claimed in claim 1 with a pharmaceutically acceptable adjuvant, diluent or carrier.

9. A compound of formula (I), or a pharmaceutically-acceptable salt or solvate thereof, as claimed in any one of claims 1 to 6 for use in therapy.

10. Use of a compound of formula (I), or a pharmaceutically-acceptable salt or solvate thereof, as claimed in any one of claims 1 to 6 in the manufacture of a medicament for use in therapy.

11. Use of a compound of formula (IA), or a pharmaceutically-acceptable salt or solvate thereof, in the manufacture of a medicament for treating a chemokine mediated disease: 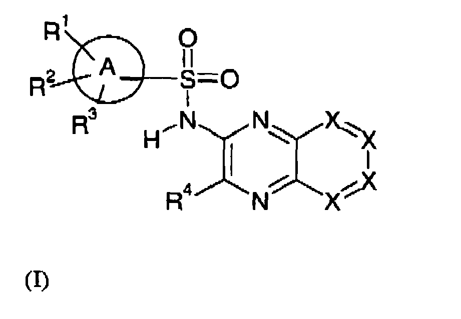 in which:
A is a thienyl or phenyl ring;
X groups are independently N or CR⁵-,
R¹, R² and R³ are independently hydrogen, halogen, cyano, CF₃, OCF₃ , OC₁₋₆ alkyl or C₁₋₆ alkyl;
R⁴ is halogen,
C₁₋₆ alkoxy where the alkyl group may be substituted with 1-3 fluorine atoms or a cyano group,
C₃₋₆ alkenyloxy or C₃₋₆ alkynyloxy where either may be optionally substituted with hydroxy or NR⁶R⁷,
OC₁₋₆ alkylR⁸;
R⁵ is independently hydrogen, C₁₋₆ alkyl where the alkyl group may be substituted with 1-3 fluorine atoms, SO₂R⁶, SO₂NR⁶R⁷, cyano, halogen, CO₂R⁹, CONR⁶R⁷; (CH₂)ₙNR⁶R⁷, (CH₂)ₙOH, C₁₋₆ alkoxy where the alkyl group may be substituted with 1-3 fluorine atoms;
R⁶ and R⁷ are independently hydrogen, C₁₋₆ alkyl, C₃₋₆ cycloalkyl or (CH₂)_{q}OH,
or R⁶ and R⁷ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy,
R⁸ is an aryl group or a 5-7 membered heteraromatic ring containing 1-4 heteroatoms selected from nitrogen, oxygen or sulphur each of which can be optionally substituted by 1-3 groups selected from halogen, C(O)NR⁶R⁷, C(C)OR⁹, hydroxy, =O, =S, CN, NO₂ NR⁶R⁷, X(CH₂)_{q}NR⁶R⁷, (CH₂)ₙNR⁶R⁷, (CH₂)ₙOH;
R⁹ is independently hydrogen or C₁₋₆ alkyl where the alkyl group may be substituted with 1-3 fluorine atoms or may form a saturated 3-6 membered ring;
Y is O or S
n is 1 ,2, 3, 4 or 5; and
q is 2, 3, 4, 5 or 6,
provided that when all X groups are independently CH, the unit AR¹R²R³ does not represent phenyl, 4-methylphenyl-, 4-fluorophenyl-, 4-chlorophenyl-, 4-ethylphenyl-, 4-propylphenyl-, 2-methylphenyl-, 3-methylphenyl-, 4-methoxyphenyl-, 4-cyanophenyl-, 4-bromophenyl- or 4-(1-methylethyl)phenyl-.

12. Use according to claim 11 in which the chemokine receptor belongs to the CCR chemokine receptor subfamily.

13. Use according to claim 11 or 12 in which the chemokine receptor is the CCR4 receptor.

14. Use according to claim 13 wherein the disease is asthma.

15. Use according to any one of claims 10 to 14 where the compound of formula (IA) is selected from:
2,3-Dichloro-N-(3-methoxyquinoxalin-2-yl)-benzenesulfonamide
N-(3-Methoxyquinoxalin-2-yl)thiophene-2-sulfonamide
N-[3-(2-Furylmethoxy)quinoxalin-2-yl]thiophene-2-sulfonamide
5-Chloro-N-(3-methoxyquinoxalin-2-yl)thiophene-2-sulfonamide
2,3-Dichloro-N-(3,7-dimethoxyquinoxalin-2-yl)benzenesulfonamide
2,3-Dichloro-N-(2-methoxypyrido[2,3-b]pyrazin-3-yl)benzenesulfonamide
2,3-Dichloro-N-(3-methoxypyrido[2,3-b]pyrazin-2-yl)benzenesulfonamide
2,3-Dichloro-N-(3,6,7-trimethoxyquinoxalin-2-yl)benzenesulfonamide
2,3-Dichloro-N-(6,7-dichloro-3-methoxyquinoxalin-2-yl)benzenesulfonamide
*N*-(7-Bromo-3-methoxypyrido[2,3-*b*]pyrazin-2-yl)-2,3-dichlorobenzenesulfonamide
*N*-(7-Bromo-2-methoxypyrido[2,3-*b*]pyrazin-3-yl)-2,3-dichlorobenzenesulfonamide
2,3-Dichloro-*N*-(3-methoxypyrido[3,4-*b*]pyrazin-2-yl)benzenesulfonamide
and pharmaceutically acceptable salts and solvates thereof.

16. A process for the preparation of a compound of formulae (I) which comprises:
(a) reaction of a compound of formula (II): 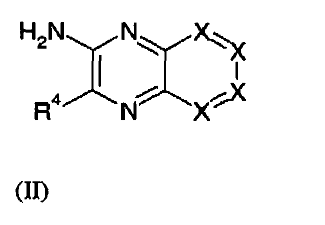 where R⁴ and X are as defined in formulae (I) or are protected derivatives thereof with a compound of formula (III): 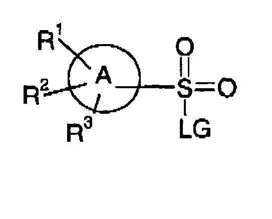 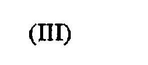 where R¹, R² and R³ are as defined in formulae (I) or are protected derivatives thereof and LG is a leaving group,
(b) reacting a compound of the formulae (IV) where LG is a leaving group 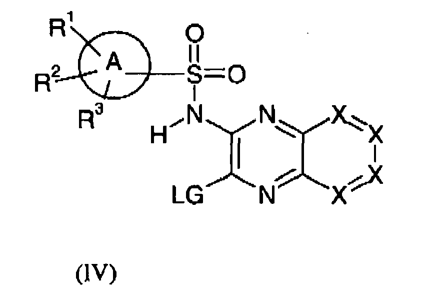 with a compound of formula (V)
HO-R¹⁰ (V)
where R¹⁰ can be C₁₋₆ alkoxy where the alkyl group may be substituted with 1-3 fluorine atoms or a cyano group;
C₃₋₆ alkenyloxy or C₁₋₆ alkynyloxy where either may be optionally substituted with hydroxy or NR⁶R⁷;
OC₁₋₆ alkylR⁸. or OC₂₋₆ alkyl-Y-R⁸
where R⁶, R⁷ and R⁸ are as defined in formulae (I) and (II),
(c) reacting a compound of formula (VI): 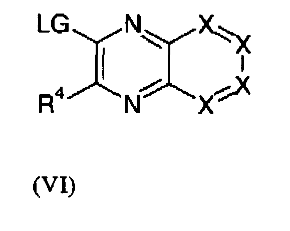
where R⁴ and X are as defined in formulae (I) or are protected derivatives thereof with a compound of formula (VII): 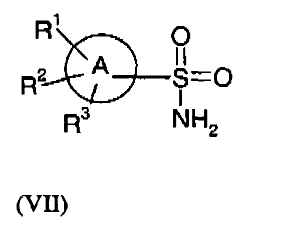 where R¹, R² and R³ are as defined in formulae (I) or are protected derivatives thereof and LG is a leaving group,
and optionally thereafter process (a), (b) or (c)
• removing any protecting groups
• converting a compound of formulae (I) to a further compound of formulae (I)
• forming a pharmaceutically acceptable salt.

## Patentansprüche

1. Verbindung der Formel (I) und pharmazeutisch annehmbare Salze oder Solvate davon: 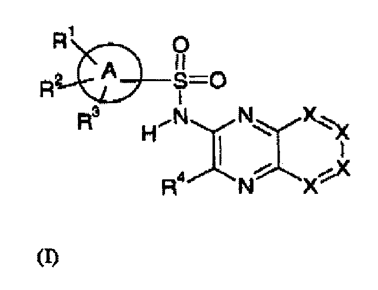 worin:
A für einen Thienyl- oder Phenylring steht;
die Gruppen X unabhängig voneinander für N oder CR⁵ stehen;
R¹, R² und R³ unabhängig voneinander für Wasserstoff, Halogen, Cyano, CF₃, OCF₃, O-C₁₋₆-Alkyl oder C₁₋₆-Alkyl stehen;
R⁴ für Halogen,
C₁₋₆-Alkoxy, wobei die Alkylgruppe durch 1 bis 3 Fluoratome oder eine Cyanogruppe substituiert sein kann,
gegebenenfalls durch Hydroxy oder NR⁶R⁷ substituiertes C₃₋₆-Alkenyloxy oder C₃₋₆-Alkinyloxy oder
O-C₁₋₆-Alkyl-R⁸ steht;
R⁵ unabhängig für Wasserstoff, C₁₋₆-Alkyl, wobei die Alkylgruppe durch 1 bis 3 Fluoratome substituiert sein kann, SO₂R⁶, SO₂NR⁶R⁷, Cyano, Halogen, CO₂R⁹, CONR⁶R⁷, (CH₂)NR⁶R⁷, (CH₂)ₙOH oder C₁₋₆-Alkoxy, wobei die Alkylgruppe durch 1 bis 3 Fluoratome substituiert sein kann, steht;
R⁶ und R⁷ unabhängig voneinander für Wasserstoff, C₁₋₆-Alkyl, C₃₋₆-Cycloalkyl oder (CH₂) qOH stehen
oder R⁶ und R⁷ gemeinsam mit dem Stickstoffatom, an das sie gebunden sind, einen 4- bis 8-gliedrigen gesättigten Ring, der 1 bis 3 unter Stickstoff, Sauerstoff und Schwefel ausgewählte Heteroatome enthält und gegebenenfalls durch C₁₋₆-Alkyl, C₁₋₆-Alkyl-OH oder Hydroxy substituiert ist, bilden,
R⁸ für eine Arylgruppe oder einen 5- bis 7-gliedrigen heteroaromatischen Ring mit 1 bis 4 unter Stickstoff, Sauerstoff und Schwefel ausgewählten Heteroatomen, jeweils gegebenenfalls substituiert durch 1 bis 3 unter Halogen, C(O)NR⁶R⁷, C(O)OR⁹, Hydroxy, =O, =S, CN, NO₂, NR⁶R⁷, X (CH₂)_{q}NR⁶R⁷, (CH₂)ₙNR⁶R⁷ oder (CH₂)ₙOH ausgewählte Gruppen, steht;
R⁹ unabhängig für Wasserstoff oder C₁₋₆-Alkyl, wobei die Alkylgruppe durch 1 bis 3 Fluoratome substituiert sein kann oder einen 3- bis 6-gliedrigen gesättigten Ring bilden kann, steht;
Y für O oder S steht;
n für 1, 2, 3, 4 oder 5 steht und
q für 2, 3, 4, 5 oder 6 steht, mit der Maßgabe, daß die folgenden Verbindungen ausgeschlossen sind:
N-(3-Methoxychinoxalin-2-yl)thiophen-2-sulfonamid und
N-[3-(2-Furylmethoxy)chinoxalin-2-yl]thiophen-2-sulfonamid,
und daß dann, wenn alle Gruppen X unabhängig voneinander für CH stehen, die Einheit AR¹R²R³ nicht für Phenyl, 4-Methylphenyl-, 4-Fluorphenyl-, 4-Chlorphenyl-, 4-Ethylphenyl-, 4-Propylphenyl-, 2-Methylphenyl-, 3-Methylphenyl-, 4-Methoxyphenyl-, 4-Cyanophenyl-, 4-Bromphenyl- oder 4-(1-Methylethyl)phenyl- steht.

2. Verbindung nach Anspruch 1, worin A für Phenyl steht.

3. Verbindung nach Anspruch 1 oder 2, worin zwei der Gruppen R¹, R² und R³ für Chlor und eine für Methyl steht.

4. Verbindung nach einem der Ansprüche 1 bis 3, worin R⁴ für C₁₋₆-Alkoxy, wobei die Alkylgruppe durch 1 bis 3 Fluoratome oder eine Cyanogruppe substituiert sein kann, oder
gegebenenfalls durch Hydroxy oder NR⁶R⁷ substituiertes C₃₋₆-Alkenyloxy oder C₃₋₆-Alkinyloxy steht.

5. Verbindung nach einem der Ansprüche 1 bis 4, worin R⁴ für C₁₋₆-Alkoxy steht.

6. Verbindung nach Anspruch 1, bei der es sich um: 2,3-Dichlor-N-(3-methoxychinoxalin-2-yl)benzolsulfonamid
5-Chlor-N-(3-methoxychinoxalin-2-yl)thiophen-2-sulfonamid
2,3-Dichlor-N-(3,7-dimethoxychinoxalin-2-yl)-benzolsulfonamid
2,3-Dichlor-N-(2-methoxypyrido[2,3-b]pyrazin-3-yl)benzolsulfonamid
2,3-Dichlor-N-(3-methoxypyrido[2,3-b]pyrazin-2-yl)benzolsulfonamid
2,3-Dichlor-N-(3,6,7-trimethoxychinoxalin-2-yl)-benzolsulfonamid
2,3-Dichlor-N-(6,7-dichlor-3-methoxychinoxalin-2-yl)benzolsulfonamid
N-(7-Brom-3-methoxypyrido[2,3-b]pyrazin-2-yl)2,3-dichlorbenzolsulfonamid
N-(7-Brom-2-methoxypyrido[2,3-b]pyrazin-3-yl)2,3-dichlorbenzolsulfonamid
2,3-Dichlor-N-(3-methoxypyrido[3,4-b]pyrazin-2-yl)benzolsulfonamid
und pharmazeutisch annehmbare Salze und Solvate davon handelt.

7. Pharmazeutische Zusammensetzung, die eine Verbindung der Formel (I) oder ein pharmazeutisch annehmbares Salz oder Solvat davon nach den Ansprüchen 1 bis 6 zusammen mit einem pharmazeutisch annehmbaren Hilfsstoff, Verdünnungsmittel oder Träger enthält.

8. Verfahren zur Herstellung einer pharmazeutischen Zusammensetzung nach Anspruch 7, bei dem man eine Verbindung der Formel (I) oder ein pharmazeutisch annehmbares Salz oder Solvat davon nach Anspruch 1 mit einem pharmazeutisch annehmbaren Hilfsstoff, Verdünnungsmittel oder Träger vermischt.

9. Verbindung der Formel (I) oder ein pharmazeutisch annehmbares Salz oder Solvat davon nach den Ansprüchen 1 bis 6 zur Verwendung bei der Therapie.

10. Verwendung einer Verbindung der Formel (I) oder eines pharmazeutisch annehmbaren Salzes oder Solvats davon nach den Ansprüchen 1 bis 6 bei der Herstellung eines Arzneimittels zur Verwendung bei der Therapie.

11. Verwendung einer Verbindung der Formel (IA) oder eines pharmazeutisch annehmbaren Salzes oder Solvats davon bei der Herstellung eines Arzneimittels zur Verwendung bei der Behandlung einer chemokinvermittelten Erkrankung: 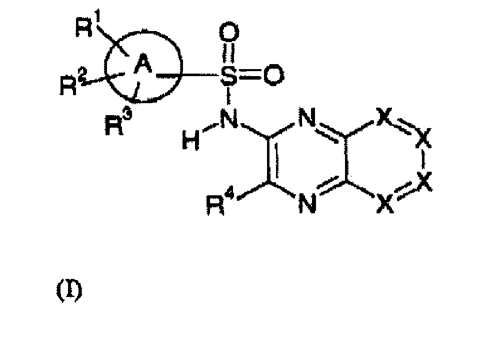 worin:
A für einen Thienyl- oder Phenylring steht;
die Gruppen X unabhängig voneinander für N oder CR⁵ stehen;
R¹, R² und R³ unabhängig voneinander für Wasserstoff, Halogen, Cyano, CF₃, OCF₃, O-C₁₋₆-Alkyl oder C₁₋₆-Alkyl stehen;
R⁴ für Halogen,
C₁₋₆-Alkoxy, wobei die Alkylgruppe durch 1 bis 3 Fluoratome oder eine Cyanogruppe substituiert sein kann,
gegebenenfalls durch Hydroxy oder NR⁶R⁷ substituiertes C₃₋₆-Alkenyloxy oder C₃₋₆-Alkinyloxy oder
O-C₁₋₆-Alkyl-R steht;
R⁵ unabhängig für Wasserstoff, C₁₋₆-Alkyl, wobei die Alkylgruppe durch 1 bis 3 Fluoratome substituiert sein kann, SO₂R⁶, SO₂NR⁶R⁷, Cyano, Halogen, CO₂R⁹, CONR⁶R⁷, (CH₂)ₙNR⁶R⁷, (CH₂)ₙOH oder C₁₋₆-Alkoxy, wobei die Alkylgruppe durch 1 bis 3 Fluoratome substituiert sein kann, steht;
R⁶ und R⁷ unabhängig voneinander für Wasserstoff, C₁₋₆-Alkyl, C₃₋₆-Cycloalkyl oder (CH₂)_{q}OH stehen
oder R⁶ und R⁷ gemeinsam mit dem Stickstoffatom, an das sie gebunden sind, einen 4- bis 8-gliedrigen gesättigten Ring, der 1 bis 3 unter Stickstoff, Sauerstoff und Schwefel ausgewählte Heteroatome enthält und gegebenenfalls durch C₁₋₆-Alkyl, C₁₋₆-Alkyl-OH oder Hydroxy substituiert ist, bilden,
R⁸ für eine Arylgruppe oder einen 5- bis 7-gliedrigen heteroaromatischen Ring mit 1 bis 4 unter Stickstoff, Sauerstoff und Schwefel ausgewählten Heteroatomen, jeweils gegebenenfalls substituiert durch 1 bis 3 unter Halogen, C(O)NR⁶R⁷, C (O) OR⁹, Hydroxy, =O, =S, CN, NO₂, NR⁶R⁷, X (CH₂)_{q}NR⁶R⁷, (CH₂)ₙNR⁶R⁷ oder (CH₂)ₙOH ausgewählte Gruppen, steht;
R⁹ unabhängig für Wasserstoff oder C₁₋₆-Alkyl, wobei die Alkylgruppe durch 1 bis 3 Fluoratome substituiert sein kann oder einen 3- bis 6-gliedrigen gesättigten Ring bilden kann, steht;
Y für 0 oder S steht;
n für 1, 2, 3, 4 oder 5 steht und
q für 2, 3, 4, 5 oder 6 steht,
mit der Maßgabe, daß dann, wenn alle Gruppen X unabhängig voneinander für CH stehen, die Einheit AR¹R²R³ nicht für Phenyl, 4-Methylphenyl-, 4-Fluorphenyl-, 4-Chlorphenyl-, 4-Ethylphenyl-, 4-Propylphenyl-, 2-Methylphenyl-, 3-Methylphenyl-, 4-Methoxyphenyl-, 4-Cyanophenyl-, 4-Bromphenyl-oder 4-(1-Methylethyl)phenyl- steht.

12. Verwendung nach Anspruch 11, bei der der Chemokinrezeptor zur Unterfamilie der CCR-Chemokinrezeptoren gehört.

13. Verwendung nach Anspruch 11 oder 12, bei der es sich bei dem Chemokinrezeptor um den CCR4-Rezeptor handelt.

14. Verwendung nach Anspruch 13, bei der es sich bei der Erkrankung um Asthma handelt.

15. Verwendung nach einem der Ansprüche 10 bis 14, wobei die Verbindung der Formel (IA) unter:
2,3-Dichlor-N-(3-methoxychinoxalin-2-yl)-benzolsulfonamid
N-(3-Methoxychinoxalin-2-yl)thiophen-2-sulfonamid
N-[3-(2-Furylmethoxy)chinoxalin-2-yl]thiophen-2-sulfonamid
5-Chlor-N-(3-methoxychinoxalin-2-yl)thiophen-2-sulfonamid
2,3-Dichlor-N-(3,7-dimethoxychinoxalin-2-yl)-benzolsulfonamid
2,3-Dichlor-N-(2-methoxypyrido[2,3-b]pyrazin-3-yl)benzolsulfonamid
2,3-Dichlor-N-(3-methoxypyrido[2,3-b]pyrazin-2-yl)-benzolsulfonamid
2,3-Dichlor-N-(3,6,7-trimethoxychinoxalin-2-yl)-benzolsulfonamid
2,3-Dichlor-N-(6,7-dichlor-3-methoxychinoxalin-2-yl)benzolsulfonamid
N-(7-Brom-3-methoxypyrido[2,3-b]pyrazin-2-yl)2,3-dichlorbenzolsulfonamid
N-(7-Brom-2-methoxypyrido[2,3-b]pyrazin-3-yl)2,3-dichlorbenzolsulfonamid
2,3-Dichlor-N-(3-methoxypyrido[3,4-b]pyrazin-2-yl)benzolsulfonamid
und pharmazeutisch annehmbaren Salzen und Solvaten davon ausgewählt ist.

16. Verfahren zur Herstellung einer Verbindung der Formel (I), bei dem man:
(a) eine Verbindung der Formel (II): 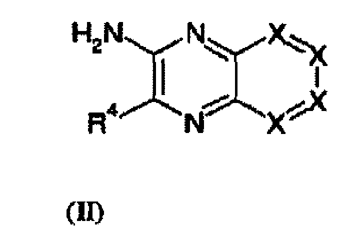 worin R⁴ und X die unter Formel (I) angegebene Bedeutung besitzen oder für geschützte Derivate davon stehen, mit einer Verbindung der Formel (III): 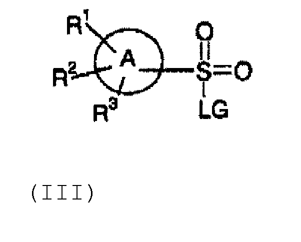 worin R¹, R² und R³ die unter Formel (I) angegebene Bedeutung besitzen oder für geschützte Derivate davon stehen und LG für eine Abgangsgruppe steht, umsetzt,
(b) eine Verbindung der Formel (IV), worin LG für eine Abgangsgruppe steht, 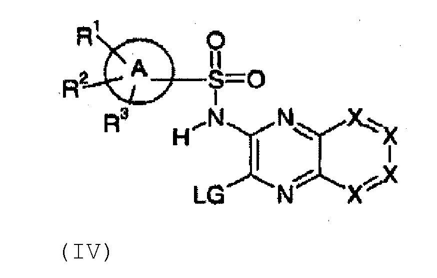 mit einer Verbindung der Formel (V)
HO-R¹⁰ (V)
worin R¹⁰ für C₁₋₆-Alkoxy, worin die Alkylgruppe durch 1 bis 3 Fluoratome oder eine Cyanogruppe substituiert sein kann; gegebenenfalls durch Hydroxy oder NR⁶R⁷ substituiertes C₃₋₆-Alkenyloxy oder C₃₋₆-Alkinyloxy; O-C₁₋₆-Alkyl-R⁸ oder O-C₂₋₆-Alkyl-Y-R⁸ steht,
wobei R⁶, R⁷ und R⁸ die unter den Formeln (I) und (II) angegebene Bedeutung besitzen, umsetzt;
(c) eine Verbindung der Formel (VI): 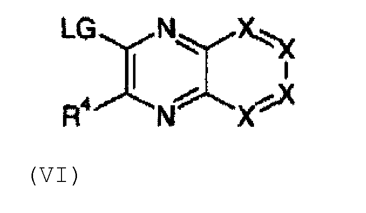
worin R⁴ und X die unter Formel (I) angegebene Bedeutung besitzen oder für geschützte Derivate davon stehen, mit einer Verbindung der Formel (VII): 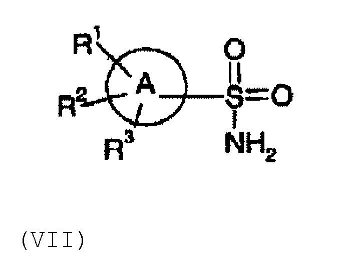 worin R¹, R² und R³ die unter Formel (I) angegebene Bedeutung besitzen oder für geschützte Derivate davon stehen und LG für eine Abgangsgruppe steht, umsetzt,
und gegebenenfalls nach (a), (b) oder (c):
• jegliche Schutzgruppen abspaltet
• eine Verbindung der Formel (I) in eine weitere Verbindung der Formel (I) umwandelt
• ein pharmazeutisch annehmbares Salz herstellt.

## Revendications

1. Composé de formule (I) et les sels ou les solvates pharmaceutiquement acceptables de celui-ci : 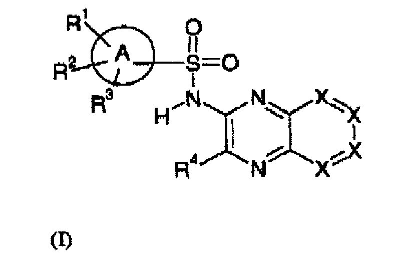 dans laquelle :
A est un cycle thiényle ou phényle ;
les groupements X sont indépendamment N ou CR⁵;
R¹, R² et R³ sont indépendamment hydrogène, halogène, cyano, CF₃, OCF₃, OC₁₋₆-alkyle ou C₁₋₆-alkyle ;
R⁴ est halogène,
C₁₋₆-alcoxy, où le groupement alkyle peut être substitué par 1-3 atomes de fluor ou un groupement cyano,
C₃₋₆-alcényloxy ou C₃₋₆-alcynyloxy, où l'un ou l'autre peut être éventuellement substitué par hydroxy ou NR⁶R⁷,
OC₁₋₆-alkyl-R⁸ ;
R⁵ est indépendamment hydrogène, C₁₋₆-alkyle, où le groupement alkyle peut être substitué par 1-3 atomes de fluor, SO₂R⁶, SO₂NR⁶R⁷, cyano, halogène, CO₂R⁹, CONR⁶R⁷ ; (CH₂)ₙNR⁶R⁷, (CH₂)ₙOH, C₁₋₆-alcoxy, où le groupement alkyle peut être substitué par 1-3 atomes de fluor ;
R⁶ et R⁷ sont indépendamment hydrogène, C₁₋₆-alkyle, C₃₋₆-cycloalkyle ou (CH₂) qOH,
ou R⁶ et R⁷, ensemble avec l'atome d'azote auquel ils sont liés, forment un cycle saturé à 4-8 chaînons contenant 1-3 hétéroatomes choisis parmi azote, oxygène et soufre, et éventuellement substitués par C₁₋₆-alkyle, C₁₋₆-alkyl-OH ou hydroxy,
R⁸ est un groupement aryle ou un cycle hétéro-aromatique à 5-7 chaînons contenant 1-4 hétéroatomes choisis parmi azote, oxygène et soufre, qui peuvent être chacun éventuellement substitués par 1-3 groupements choisis parmi halogène, C(O)NR⁶R⁷, C(O)OR⁹, hydroxy, =O, =S, CN, NO₂, NR⁶R⁷, X(CH₂)_{q}NR⁶R⁷, (CH₂)ₙNR⁶R⁷ ou (CH₂)ₙOH ;
R⁹ est indépendamment hydrogène ou C₁₋₆-alkyle, où le groupement alkyle peut être substitué par 1-3 atomes de fluor ou peut former un cycle saturé à 3-6 chaînons ;
Y est 0 ou S,
n est 1, 2, 3, 4 ou 5 ; et
q est 2, 3, 4, 5 ou 6, à condition que les composés suivants soient exclus :
le N-(3-méthoxyquinoxalin-2-yl)thiophène-2-sulfonamide, et
le N-[3-(2-furylméthoxy)quinoxalin-2-yl]thiophène-2-sulfonamide,
et que, lorsque tous les groupements X sont indépendamment CH, le motif AR¹R²R³ ne représente pas phényle, 4-méthylphényl-, 4-fluorophényl-, 4-chloro-phényl-, 4-éthylphényl-, 4-propylphényl-, 2-méthylphényl-, 3-méthylphényl-, 4-méthoxyphényl-, 4-cyanophényl-, 4-bromophényl- ou 4-(1-méthyléthyl)-phényl-.

2. Composé selon la revendication 1, **caractérisé en ce que** A est phényle.

3. Composé selon la revendication 1 ou 2, **caractérisé en ce que** deux de R¹, R² et R³ sont chloro ou l'un est méthyle.

4. Composé selon l'une quelconque des revendications 1 à 3, **caractérisé en ce que** R⁴ est
C₁₋₆-alcoxy, où le groupement alkyle peut être substitué par 1-3 atomes de fluor ou un groupement cyano, ou
C₃₋₆-alcényloxy ou C₃-₆-alcynyloxy, où l'un ou l'autre peut être éventuellement substitué par hydroxy ou NR⁶R⁷.

5. Composé selon l'une quelconque des revendications 1 à 4, **caractérisé en ce que** R⁴ est C₁₋₆-alcoxy.

6. Composé selon la revendication 1, **caractérisé en ce qu'**il s'agit de :
le 2,3-dichloro-N-(3-méthoxyquinoxalin-2-yl)benzène-sulfonamide,
le 5-chloro-N-(3-méthoxyquinoxalin-2-yl)thiophène-2-sulfonamide,
le 2,3-dichloro-N-(3,7-diméthoxyquinoxalin-2-yl)-benzènesulfonamide,
le 2,3-dichloro-N-(2-méthoxypyrido[2,3-b]pyrazin-3-yl)benzènesulfonamide,
le 2,3-dichloro-N-(3-méthoxypyrido[2,3-b]pyrazin-2-yl)benzènesulfonamide,
le 2,3-dichloro-N-(3,6,7-triméthoxyquinoxalin-2-yl)-benzènesulfonamide,
le 2,3-dichloro-N-(6,7-dichloro-3-méthoxyquinoxalin-2-yl)benzènesulfonamide,
le *N*-(7-bromo-3-méthoxypyrido[2,3-*b*]pyrazin-2-yl)-2,3-dichlorobenzènesulfonamide,
le *N*-(7-bromo-2-méthoxypyrido[2,3-*b*]pyrazin-3-yl)-2,3-dichlorobenzènesulfonamide,
le 2,3-dichloro-*N*-(3-méthoxypyrido[3,4-*b*]pyrazin-2-yl)benzènesulfonamide,
et les sels et les solvates pharmaceutiquement acceptables de ceux-ci.

7. Composition pharmaceutique comprenant un composé de formule (I), ou un sel ou un solvate pharmaceutiquement acceptable de celui-ci, selon les revendications 1 à 6, en association avec un adjuvant, un diluant ou un support pharmaceutiquement acceptable.

8. Procédé de préparation d'une composition pharmaceutique selon la revendication 7, **caractérisé en ce qu'**il comprend le mélange d'un composé de formule (I), ou d'un sel ou d'un solvate pharmaceutiquement acceptable de celui-ci, selon la revendication 1, avec un adjuvant, un diluant ou un support pharmaceutiquement acceptable.

9. Composé de formule (I), ou un sel ou un solvate pharmaceutiquement acceptable de celui-ci, selon l'une quelconque des revendications 1 à 6, destiné à être utilisé en thérapie.

10. Utilisation d'un composé de formule (I), ou un sel ou un solvate pharmaceutiquement acceptable de celui-ci, selon l'une quelconque des revendications 1 à 6, dans la fabrication d'un médicament destiné à être utilisé en thérapie.

11. Utilisation d'un composé de formule (IA), ou d'un sel ou d'un solvate pharmaceutiquement acceptable de celui-ci, dans la fabrication d'un médicament destiné à traiter une maladie médiée par une chimiokine : 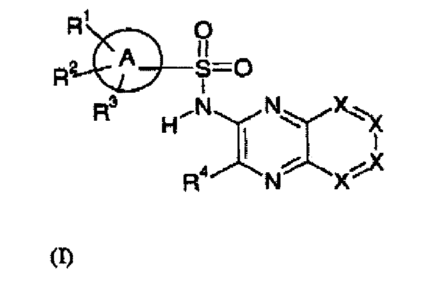 dans laquelle :
A est un cycle thiényle ou phényle ;
les groupements X sont indépendamment N ou CR⁵ ;
R¹, R² et R³ sont indépendamment hydrogène, halogène, cyano, CF₃, OCF₃, OC₁₋₆-alkyle ou C₁₋₆-alkyle ;
R⁴ est halogène,
C₁₋₆-alcoxy, où le groupement alkyle peut être substitué par 1-3 atomes de fluor ou un groupement cyano,
C₃₋₆-alcényloxy ou C₃₋₆-alcynyloxy, où l'un ou l'autre peut être éventuellement substitué par hydroxy ou NR⁶R⁷ ,
OC₁₋₆-alkyl-R⁸ ;
R⁵ est indépendamment hydrogène, C₁₋₆-alkyle, où le groupement alkyle peut être substitué par 1-3 atomes de fluor, SO₂R⁶, SO₂NR⁶R⁷, cyano, halogène, CO₂R⁹, CONR⁶R⁷ ; (CH₂)nNR⁶R⁷, (CH₂)nOH, C₁₋₆-alcoxy, où le groupement alkyle peut être substitué par 1-3 atomes de fluor ;
R⁶ et R⁷ sont indépendamment hydrogène, C₁₋₆-alkyle, C₃₋₆-cycloalkyle ou (CH₂)qOH,
ou R⁶ et R⁷, ensemble avec l'atome d'azote auquel ils sont liés, forment un cycle saturé à 4-8 chaînons contenant 1-3 hétéroatomes choisis parmi azote, oxygène et soufre, et éventuellement substitués par C₁₋₆-alkyle, C₁₋₆-alkyl-OH ou hydroxy,
R⁸ est un groupement aryle ou un cycle hétéro-aromatique à 5-7 chaînons contenant 1-4 hétéroatomes choisis parmi azote, oxygène et soufre, qui peuvent être chacun éventuellement substitués par 1-3 groupements choisis parmi halogène, C(O)NR⁶R⁷, C(O)OR⁹, hydroxy, =O, =S, CN, NO₂, NR⁶R⁷, X(CH₂)qNR⁶R⁷, (CH₂)nNR⁶R⁷ ou (CH₂)nOH ;
R⁹ est indépendamment hydrogène ou C₁₋₆-alkyle, où le groupement alkyle peut être substitué par 1-3 atomes de fluor ou peut former un cycle saturé à 3-6 chaînons ;
Y est 0 ou S ;
n est 1, 2, 3, 4 ou 5 ; et
q est 2, 3, 4, 5 ou 6,
à condition que, lorsque tous les groupements X sont indépendamment CH, le motif AR¹R²R³ ne représente pas phényle, 4-méthylphényl-, 4-fluorophényl-, 4-chloro-phényl-, 4-éthylphényl-, 4-propylphényl-, 2-méthylphényl-, 3-méthylphényl-, 4-méthoxyphényl-, 4-cyanophényl-, 4-bromophényl- ou 4-(1-méthyléthyl)-phényl-.

12. Utilisation selon la revendication 11, **caractérisée en ce que** le récepteur de chimiokine appartient à la sous-famille de récepteurs de chimiokine CCR.

13. Utilisation selon la revendication 11 ou 12, **caractérisée en ce que** le récepteur de chimiokine est le récepteur CCR4.

14. Utilisation selon la revendication 13, **caractérisée en ce que** la maladie est l'asthme.

15. Utilisation selon l'une quelconque des revendications 10 à 14, **caractérisée en ce que** le composé de formule (IA) est choisi parmi :
le 2,3-dichloro-N-(3-méthoxyquinoxalin-2-yl)benzène-sulfonamide,
le N-(3-méthoxyquinoxalin-2-yl)thiophène-2-sulfonamide,
le N-[3-(2-furylméthoxy)quinoxalin-2-yl]thiophène-2-sulfonamide,
le 5-chloro-N-(3-méthoxyquinoxalin-2-yl)thiophène-2-sulfonamide,
le 2,3-dichloro-N-(3,7-diméthoxyquinoxalin-2-yl)-benzènesulfonamide,
le 2,3-dichloro-N-(2-méthoxypyrido[2,3-b]pyrazin-3-yl)benzènesulfonamide,
le 2,3-dichloro-N-(3-méthoxypyrido[2,3-b]pyrazin-2-yl)benzènesulfonamide,
le 2,3-dichloro-N-(3,6,7-triméthoxyquinoxalin-2-yl)-benzènesulfonamide,
le 2,3-dichloro-N-(6,7-dichloro-3-méthoxyquinoxalin-2-yl)benzènesulfonamide,
le *N*-(7-bromo-3-méthoxypyrido[2,3-*b*]pyrazin-2-yl)-2,3-dichlorobenzènesulfonamide,
le *N*-(7-bromo-2-méthoxypyrido[2,3-*b*]pyrazin-3-yl)-2,3-dichlorobenzènesulfonamide,
le 2,3-dichloro-*N*-(3-méthoxypyrido[3,4-*b*]pyrazin-2-yl)benzènesulfonamide,
et les sels et les solvates pharmaceutiquement acceptables de ceux-ci.

16. Procédé de préparation d'un composé de formule (I), **caractérisé en ce qu'**il comprend :
(a) la réaction d'un composé de formule (II) : 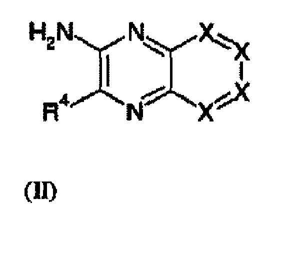 dans laquelle R⁴ et X sont tels que définis dans la formule (I) ou sont des dérivés protégés de ceux-ci, avec un composé de formule (III) : 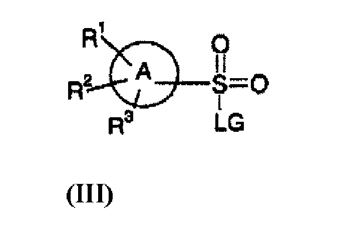 dans laquelle R¹ R² et R³ sont tels que définis dans la formule (I) ou sont des dérivés protégés de ceux-ci et LG est un groupement partant,
(b) la réaction d'un composé de formule (IV), dans laquelle LG est un groupement partant, 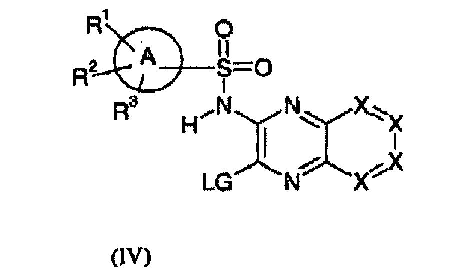 avec un composé de formule (V)
**HO-R¹⁰** **(V)**
dans laquelle R¹⁰ peut être C₁₋₆-alcoxy, où le groupement alkyle peut être substitué par 1-3 atomes de fluor ou un groupement cyano ;
C₃₋₆-alcényloxy ou C₃₋₆-alcynyloxy, où l'un ou l'autre peut être éventuellement substitué par hydroxy ou NR⁶R⁷ ;
OC₁₋₆-alkyl-R⁸ ou OC₂₋₆-alkyl-Y-R⁸,
où R⁶, R⁷ et R⁸ sont tels que définis dans les formules (I) et (II),
(c) la réaction d'un composé de formule (VI) : 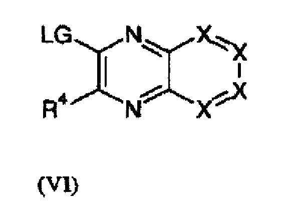
dans laquelle R⁴ et X sont tels que définis dans la formule (I) ou sont des dérivés protégés de ceux-ci, avec un composé de formule (VII) : 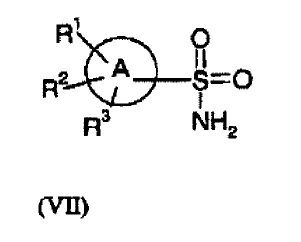 dans laquelle R¹, R² et R³ sont tels que définis dans la formule (I) ou sont des dérivés protégés de ceux-ci et LG est un groupement partant,
et, éventuellement après le procédé (a), (b) ou (c) :
• l'élimination de tous les groupements protecteurs,
• la transformation d'un composé de formule (I) en un autre composé de formule (I),
• la formation d'un sel pharmaceutiquement acceptable.
